# EUROPEAN PATENT APPLICATION

(11) **EP 4 846 964 A1**
(43) Date of publication of application: **02.12.2026**
(21) Application number: 25744486.9
(22) Date of filing: 15.01.2025
(51) Int. Cl.: A61B 18/12, A61B 17/32

(54) **METHOD FOR DETERMINING ABLATION ENDPOINT OF INTRAVASCULAR NERVE ABLATION AND ABLATION DEVICE**

(30) Priority: 26.01.2024 CN 202410113824
(71) Applicant: Brattea Medtech Co., Ltd., Shanghai 201114 (CN)
(72) Inventor: GUO, Jiulin, Shanghai 200231 (CN); HUANG, Hanli, Shanghai 200231 (CN); WANG, Yan, Shanghai 200231 (CN)
(74) Representative: Wang, Bo
(86) International application number: PCT/CN2025/072405
(87) International publication number: WO 2025/157043

(57) **Abstract**

A method for determining an ablation endpoint of an intravascular nerve ablation, and an ablation device. The determination method comprises the following steps: setting target values and target change ranges of operating parameters, wherein the operating parameters at least include power, temperature, impedance, related operating parameters, ablation duration and the like; sending an ablation catheter to a target ablation position and confirming good electrode contact; periodically collecting operating parameter data at each electrode at set time intervals; and performing corresponding calculation processing on each round of collected data to obtain related processing data such as power, temperature, impedance difference, growth rate, maximum value, minimum value, maximum fluctuation range, and standard deviation; and comprehensively determining an ablation endpoint on the basis of collected data of the power, temperature, impedance, and ablation duration, and processed data of the power, temperature, and impedance.

## Description

### BACKGROUND

### Technical Field

The present disclosure relates to a method for determining an ablation endpoint, further relates to a radio frequency ablation device using the determination method, and belongs to the technical field of medical devices.

### Related Art

Intravascular nerve ablation refers to a medical means of placing an ablation catheter into a designated location in a body by using an interventional operation, and destroying a lesion nerve tissue around a blood vessel in a target area by using radio frequency energy, to achieve an objective of ablation. Usually, the intravascular nerve ablation may be used for treatment of some diseases related to neural activity, such as hypertension and diabetes.

In the conventional art, in the intravascular nerve ablation, an ablation endpoint needs to be determined by using manual control or relying on a single means such as total output energy, and a single-point tissue impedance variation and temperature variation, to terminate an ablation operation. This method cannot accurately determine a specific ablation progress and an ablation effect, and therefore cannot accurately determine an ablation endpoint. In another aspect, single-point impedance measurement is not comprehensive enough to evaluate vascular damage, and cannot completely avoid vasospasm that may occur during an ablation operation and a series of symptoms that may occur after the operation. Therefore, it is necessary to provide a multi-parameter comprehensive determination method for a nerve ablation endpoint.

### SUMMARY

A primary technical problem to be solved by the present disclosure is to provide a method for determining an ablation endpoint of an intravascular nerve ablation.

Another technical problem to be solved by the present disclosure is to provide an ablation device using the determination method.

To achieve the above objectives, the present disclosure adopts the following technical solutions:
According to a first aspect of embodiments of the present disclosure, a method for determining an ablation endpoint of an intravascular nerve ablation is provided, including the following steps:
(1) setting target values and target variation ranges of operating parameters, where the operating parameters at least include a power, a temperature, an impedance, and an ablation duration, related operating parameters of the power, the temperature, and the impedance, weights of the operating parameters, a total valid value threshold, a qualified count threshold, and a continuous qualified count threshold;
(2) sending an ablation catheter to a target ablation position and confirming good electrode contact;
(3) cyclically collecting operating parameter data at each electrode at set time intervals, including data of the power, the temperature, the impedance, and the ablation duration;
(4) performing corresponding calculation processing on each round of collected data from a second round of collected data, to obtain related processed data of the power, the temperature, and the impedance at each electrode, where the related processed data includes differences, maximum values, minimum values, maximum fluctuation ranges, and standard deviations of the power, the temperature, and the impedance, and increase rates of the power, the temperature, and the impedance when reaching the set target variation ranges for the first time; and
(5) determining an ablation endpoint according to the collected data of the power, the temperature, the impedance, and the ablation duration, and the processed data of the power, the temperature, and the impedance.

Preferably, in step (1), the related operating parameters include differences and increase rates between radio frequency powers, ablation temperatures, and tissue impedances in data continuously collected in two rounds, and a maximum value, a minimum value, and a standard deviation value of the radio frequency power, the ablation temperature, and the tissue impedance in currently collected data.

Preferably, step (3) includes the following substeps:
(31) in a monitoring mode, collecting impedance data between each electrode and a body surface neutral electrode, collecting impedance data between every two electrodes, and collecting temperature data at each electrode; and
(32) in a radio frequency mode, collecting the power data and the ablation duration data output to each electrode.

Preferably, in step (4), a power difference *Pdᵢⱼ,* a temperature difference *Tdᵢⱼ,* and impedance differences *Zdᵢⱼ* and *Zdᵢᵢⱼ* between each round of collected data a previous round of collected data are calculated as follows: ${Pd}_{ij} = {P}_{ij} - {P}_{i \left(j-1\right)}$ ${Td}_{ij} = {T}_{ij} - {T}_{i \left(j-1\right)}$ ${Zd}_{ij} = {Z}_{ij} - {Z}_{i \left(j-1\right)}$ ${Zd}_{iij} = {Z}_{iij} - {Z}_{ii \left(j-1\right)}$ where j represents a j^{th} round of data collection, and j-1 represents a previous round of data collection; *Pij* and *Tij* represent power data and temperature data in the j^{th} round of data collection, and *Zij* and *Ziij* represent impedance data in the j^{th} round of data collection; *P*_{*i*(*j*-1)} and *T*_{*i*(*j*-1)} represent power data and temperature data in a (j-1)^{th} round of data collection, and *Z*_{*i*(*j*-1)} and *Z*_{*ii*(*j*-1)} represent impedance data in the (j-1)^{th} round of data collection; and *i* represents an electrode number, and *ii* represents a number between two electrodes.

Preferably, in step (4), after each round of collected data, whether power data, temperature data, and impedance data all reach the set target variation ranges for the first time is determined; increase rates of the power data, the temperature data, and the impedance data are calculated if the power data, the temperature data, and the impedance data all reach the set target variation ranges for the first time; a next step is skipped if the power data, the temperature data, and the impedance data do not simultaneously reach the set target variation ranges or do not reach the set target variation ranges for the first time;
after a j^{th} round of collected data, and when the power, the temperature, and the impedance all reach the set target variation ranges, a power increase rate *Pinᵢⱼ,* a temperature increase rate *Tinᵢⱼ,* and impedance increase rates *Zinᵢⱼ* and *Zinᵢᵢⱼ* are calculated as follows: ${Pin}_{ij} = \frac{\left({P}_{ij}-{P}_{is}\right)}{{t}_{j}}$ ${Tin}_{ij} = \frac{\left({T}_{ij}-{T}_{is}\right)}{{t}_{j}}$ ${Zin}_{ij} = \frac{\left({Z}_{ij}-{Z}_{is}\right)}{{t}_{j}}$ ${Zin}_{iij} = \frac{\left({Z}_{iij}-{Z}_{iis}\right)}{{t}_{j}}$
where j represents a j^{th} round of data collection, *Pij* and *Tij* represent the power data and the temperature data in the j^{th} round of data collection, *Zij* and *Ziij* represent the impedance data in the j^{th} round of data collection, and *tⱼ* is an ablation duration in the j^{th} round of data collection; *i* represents an electrode number, and *ii* represents a number between two electrodes; and *Pᵢₛ* and *Tᵢₛ* represent an initial power value and an initial temperature value obtained in first data collection, and *Zᵢₛ* and *Zᵢᵢₛ* represent initial impedance values obtained in the first data collection.

Preferably, in step (4), after each round of collected data, a maximum value *Pmaxᵢⱼ,* a minimum value *Pminᵢⱼ,* and a maximum fluctuation range *PDᵢⱼ* of the power in a current ablation process are calculated as follows: ${Pmax}_{ij} = {max}_{n = 1 , 2 … j} {P}_{in}$ ${Pmin}_{ij} = {min}_{n = 1 , 2 … j} {P}_{in}$ ${PD}_{ij} = {Pmax}_{ij} - {Pmin}_{ij}$
after each round of collected data, a maximum value *Tmaxᵢⱼ,* a minimum value *Tminᵢⱼ,* and a maximum fluctuation range *TDᵢⱼ* of the temperature in the current ablation process are calculated as follows: ${Tmax}_{ij} = {max}_{n = 1 , 2 … j} {T}_{in}$ ${Tmin}_{ij} = {min}_{n = 1 , 2 … j} {T}_{in}$ ${TD}_{ij} = {Tmax}_{ij} - {Tmin}_{ij}$
after each round of collected data, maximum values *Zmaxᵢⱼ* and *Zmaxᵢᵢⱼ,* minimum values *Zminᵢⱼ* and *Zminᵢᵢⱼ,* and maximum fluctuation ranges *ZDᵢⱼ* and *ZDᵢᵢⱼ* of the impedance in the current ablation process are calculated as follows: ${Zmax}_{ij} = {max}_{n = 1 , 2 … j} {Z}_{in}$ ${Zmax}_{iij} = {max}_{n = 1 , 2 … j} {Z}_{iin}$ ${Zmin}_{ij} = {min}_{n = 1 , 2 … j} {Z}_{in}$ ${Zmin}_{iij} = {min}_{n = 1 , 2 … j} {Z}_{iin}$ ${ZD}_{ij} = {Zmax}_{ij} - {Zmin}_{ij}$ ${ZD}_{iij} = {Zmax}_{iij} - {Zmin}_{iij}$
where j represents a j^{th} round of data collection, n represents a sequence number of each round of collected data, *Pᵢₙ* represents a power value in data collected each time, *Tᵢₙ* represents a temperature value in the data collected each time, *Zᵢₙ* and *Zᵢᵢₙ* represent impedance values collected each time, *i* represents an electrode number, and *ii* represents a number between two electrodes.

Preferably, in step (4), after each round of data collection, a power standard deviation *Pstdᵢⱼ,* a temperature standard deviation *Tstdᵢⱼ,* and impedance standard deviations *Zstdᵢⱼ* and *Zstdᵢᵢⱼ* in a current ablation process are calculated as follows: ${Pstd}_{ij} = {std}_{n = 1 , 2 … j} {P}_{in}$ ${Tstd}_{ij} = {std}_{n = 1 , 2 … j} {T}_{in}$ ${Zstd}_{ij} = {std}_{n = 1 , 2 … j} {Z}_{in}$ ${Zstd}_{iij} = {std}_{n = 1 , 2 … j} {Z}_{iin}$ where *j* represents a *j^{th}* round of data collection, *n* represents a sequence number of each round of collected data, *Pᵢₙ* represents a power value in data collected each time, *Tᵢₙ* represents a temperature value in the data collected each time, *Zᵢₙ* and *Zᵢᵢₙ* represents impedance values collected each time, *i* represents an electrode number, and *ii* represents a number between two electrodes.

Preferably, step (5) includes the following substeps:
(51) calculating a valid value *r_{k- j}* of each piece of parameter data after each round of collected data: ${r}_{k - j} = \left\{\begin{matrix}0 , & {X}_{k - j} < {Y}_{k} \\ 1 , & {X}_{k - j} \geq {Y}_{k}\end{matrix}\right.$
where *j* represents a *j^{th}* round of data collection, *k* represents numbers of collected data and processed data obtained by one round of data collection, k=1, 2, 3, ..., *Xₖ₋ⱼ* represents specific collected data or processed data, *ωₖ* represents a set weight corresponding to the specific collected data or processed data, and *Yₖ* represents a set target value corresponding to the specific collected data or processed data;
(52) calculating a total valid value *Nⱼ*: ${N}_{j} = {\sum }_{k = 1}^{232} {ω}_{k} \times {r}_{k - j}$
where 232 is a total quantity of the collected data and processed data obtained in one round of data collection; and
(53) accumulating a total qualified count Q and a maximum value Q1 of continuous qualified counts:
   comparing the total valid value after each round of data collection with a set total valid value threshold, denoting a current data collection as a qualified count when the total valid value after the data collection is greater than or equal to the total valid value threshold, and accumulating the qualified count in the total qualified count (Q); and simultaneously, recording the maximum value (Q1) of the continuous qualified counts.

Preferably, when the total qualified count obtained after one round of data collection reaches a set qualified count threshold, or the maximum value of the continuous qualified counts reaches a set continuous qualified count threshold, it is determined that a current ablation reaches an endpoint.

According to a second aspect of the embodiments of the present disclosure, a radio frequency ablation device is provided, including an ablation catheter, an operating handle, and a radio frequency ablation host, where the ablation catheter, the operating handle, and the radio frequency ablation host are sequentially connected, where
a plurality of electrodes and a plurality of temperature sensing elements are disposed at a front end of the ablation catheter;
the radio frequency ablation host at least includes a radio frequency signal generation module and an ablation control module; and
the ablation control module is configured to control an entire ablation process and an ablation endpoint, the ablation control module includes a processor and a memory, and the processor is coupled to the memory, where the memory is configured to store a computer program, and the processor is configured to execute the computer program stored in the memory, to perform the foregoing method for determining an ablation endpoint of an intravascular nerve ablation.

Compared with the conventional art, according to the method for determining an ablation endpoint of an intravascular nerve ablation provided in the present disclosure, operating parameters of a power, a temperature, an impedance, and an ablation duration at each electrode are cyclically collected, and a plurality of related operating parameters of the power, the temperature, and the impedance are obtained through data processing, to accurately and comprehensively determine an ablation progress and an ablation endpoint, thereby ensuring an ablation effect and ablation safety. Therefore, the method for determining an ablation endpoint of an intravascular nerve ablation according to the present disclosure has beneficial effects of safety, accuracy, optimization of an ablation effect, and the like.

### BRIEF DESCRIPTION OF THE DRAWINGS

FIG. 1 is a working schematic diagram of a typical radio frequency ablation device in the conventional art;
FIG. 2 is a schematic structural diagram of a deployment of an ablation catheter and an electrode in the foregoing radio frequency ablation device;
FIG. 3 is a schematic diagram of variation curves of a power, a temperature, and an impedance in a radio frequency ablation process according to an embodiment of the present disclosure;
FIG. 4 is a flowchart of a method for determining an ablation endpoint of an intravascular nerve ablation according to an embodiment of the present disclosure; and
FIG. 5 is a schematic structural diagram of a radio frequency ablation device according to an embodiment of the present disclosure.

### DETAILED DESCRIPTION

The following describes technical content of the present disclosure in detail with reference to drawings and specific embodiments.

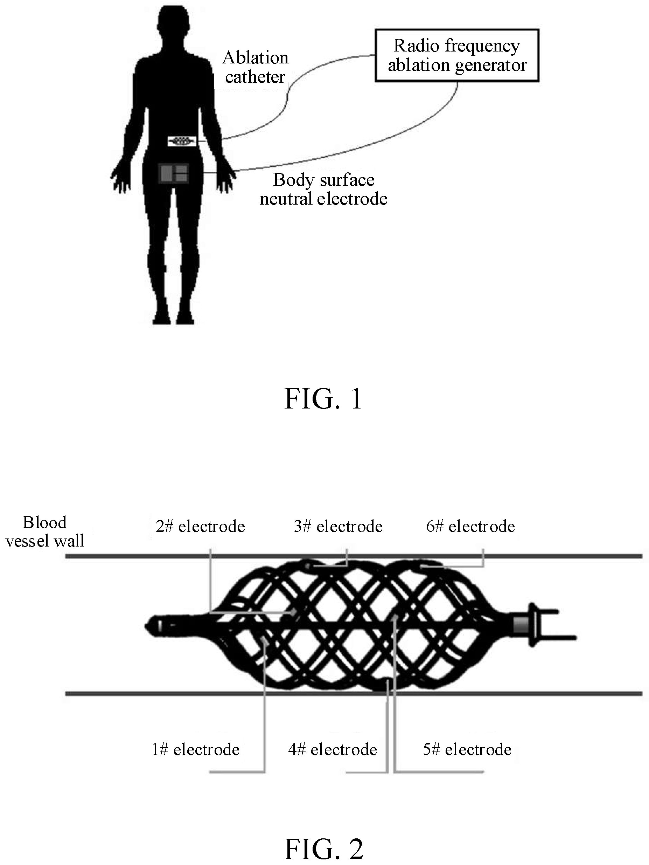

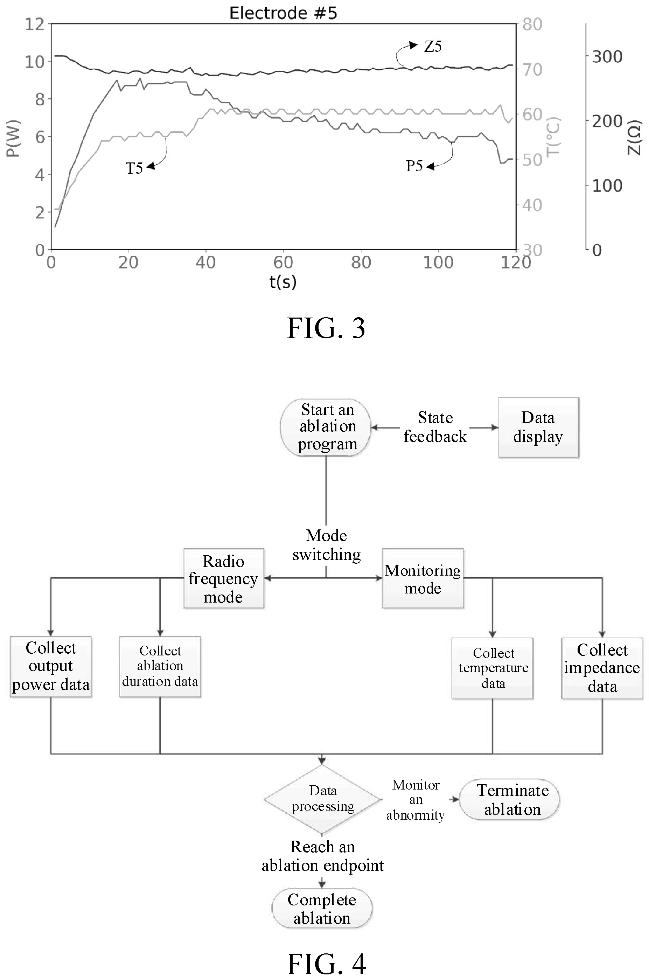

As shown in FIG. 1 and FIG. 2, in a working process of a typical radio frequency ablation device, a radio frequency ablation generator is configured to generate a radio frequency micro-current, and provide the radio frequency micro-current to an ablation catheter, to control an entire ablation process and an ablation endpoint. The ablation catheter is placed to a designated location in a body by using an interventional operation. The catheter is in a basket-like cylindrical shape after being opened. Six electrodes are disposed on the basket-like cylindrical catheter, and cooperate with a body surface neutral electrode for detecting tissue impedance in an ablation area. Simultaneously, a temperature sensing element is further disposed at each electrode. In an ablation process, a system needs to detect changes in parameters of power, temperature, and impedance at each electrode in real time, to control an ablation progress and determine an ablation endpoint. FIG. 3 are variation curves of a power P5, a temperature T5, and an impedance Z5 at an electrode 5 with an ablation duration t.

As shown in FIG. 4, an embodiment of the present disclosure provides a method for determining an ablation endpoint of an intravascular nerve ablation, including the following steps:
S1: Set target values and target variation ranges of operating parameters. The operating parameters at least include a radio frequency power, an ablation temperature, a tissue impedance, and an ablation duration, and related operating parameters of the radio frequency power, the ablation temperature, and the tissue impedance, and further include weights of the foregoing parameters, a total valid value threshold, a qualified count threshold, and a continuous qualified count threshold. The related operating parameters include differences and increase rates between radio frequency powers, ablation temperatures, and tissue impedances in data continuously collected in two rounds, and a maximum value, a minimum value, and a standard deviation value of the radio frequency power, the ablation temperature, and the tissue impedance in currently collected data.

The radio frequency power refers to a radio frequency signal power output to each electrode in an ablation process, and is briefly referred to as a power below. A target value of the radio frequency power is P, and a target variation range of the radio frequency power is [P_{L}, P_{H}]. The radio frequency power is an important operating parameter affecting an ablation effect and ablation safety, and target values of the radio frequency power are set to be different in different ablation areas. In an ablation process, a system dynamically adjusts magnitude of the radio frequency power within a power target variation range according to temperature data.

The ablation temperature refers to a tissue temperature of at each electrode in an ablation process, and is briefly referred to as a temperature below. A target value of the ablation temperature is T, and a target variation range of the ablation temperature is [T_{L}, T_{H}]. The ablation temperature is alternatively an important operating parameter affecting an ablation effect and ablation safety. In consideration of safety, a set value of the ablation temperature is usually 60°C. At the ablation temperature, nerve cells are irreversibly destroyed after a period of time.

The tissue impedance refers to a tissue impedance between each electrode and a body surface neutral electrode and a tissue impedance between every two electrodes, and is briefly referred to as an impedance below. A target value of the tissue impedance is Z, and a target variation range of the tissue impedance is [Z_{L}, Z_{H}]. In a normal ablation process, as a nerve is destroyed by a high temperature, a tissue impedance value gradually decreases. Therefore, a tissue state and an ablation effect in a nerve ablation process can be monitored by detecting a tissue impedance value in real time. When an abnormality occurs in a variation of the impedance value, it indicates that a problem occurs in electrode contact in the ablation process, and the system needs to stop ablating or perform adjustment processing. By monitoring variations in impedance values among electrodes, variations of nerve tissues between every two electrodes in an ablation process can be more comprehensively evaluated, and an ablation effect can be evaluated in a more detailed manner, thereby ensuring that ablation is completed without damaging a blood vessel wall.

S2: Send an ablation catheter to a target ablation position and confirm good electrode contact.

The electrode contact is determined by using an impedance value fed back by each electrode, and impedance value data and the like may be displayed and confirmed by using a display screen. Usually, when the impedance value is within a range of 50 Ohm to 500 Ohm, it is confirmed that an electrode contact state is good. When the impedance value exceeds the range, it is determined that the electrode contact is abnormal, and the system issues a warning prompt.

S3: Cyclically collect operating parameter data at each electrode, including power data, temperature data, impedance data, and ablation duration data. Each piece of parameter data collected for the first time is referred to as an initial value, and cyclic data collection is performed at set time intervals after the first collection. In an embodiment of the present disclosure, a set time interval is 0.5 s. A specific process of each data collection is as follows:
S31: In a monitoring mode, collect impedance data between each electrode and a body surface neutral electrode as first impedance data, collect impedance data between every two electrodes as second impedance data, and collect temperature data at each electrode.

During each data collection, there are six electrodes in total on a catheter, which are represented by a number i, where i=1, 2, 3, 4, 5, or 6, the number between two of the electrodes is represented by ii, and there are 15 numbers in total, for example, 12, 13, 14, and 23, 24, 25, so six pieces of temperature data in total are obtained in each data collection; and there are six pieces of first impedance data in total, and 15 pieces of second impedance data in total, 21 pieces of impedance data in total are obtained by adding the first impedance data and the second impedance data.

S32: In a radio frequency mode, collect power data and ablation duration data output to each electrode.

During each data collection, there are six pieces of power data in total, and one piece of ablation duration data in total. Therefore, 34 pieces of data are obtained in each collection above, which are referred to one round of collected data. The collected data is stored in real time and is transmitted to a data processing module for performing corresponding data processing.

S4: Perform corresponding calculation processing on each round of collected data from a second round of collected data, to obtain related processed data of the power, the temperature, and the impedance at each electrode. The related processed data includes differences, maximum values, minimum values, maximum fluctuation ranges, and standard deviations of the power, the temperature, and the impedance, and increase rates of the power, the temperature, and the impedance when reaching the set target variation ranges for the first time.

S41: Calculate differences corresponding to the power data, the temperature data, and the impedance data between each round of collected data and a previous round of collected data, to obtain variation values of the parameters.

It is assumed that in a j^{th} round of collected data, the power data is Pij, the temperature data is Tij, and the impedance data is Zij and Ziij, where i represents an electrode number 1, 2, 3, 4, 5, or 6, and Zii represents impedance data between the every two of the 15 electrodes. Differences between the parameters are calculated as follows: ${Pd}_{ij} = {P}_{ij} - {P}_{i \left(j-1\right)}$ (1)
where *Pdᵢⱼ* is a difference between a power collected in a j^{th} round and a power collected in a (j-1)^{th} round; and *P*_{*i*(*j*-1)} is a power value collected in the (j-1)^{th} round. ${Td}_{ij} = {T}_{ij} - {T}_{i \left(j-1\right)}$ (2)
where *Tdᵢⱼ* is a difference between a temperature collected in the j^{th} round and a temperature collected in the (j-1)^{th} round; and *T*_{*i*(*j*-1)} is a temperature value collected in the (j-1)^{th} round. ${Zd}_{ij} = {Z}_{ij} - {Z}_{i \left(j-1\right)} , and {Zd}_{iij} = {Z}_{iij} - {Z}_{ii \left(j-1\right)}$ (3)
where *Zdᵢⱼ* and *Zdᵢᵢⱼ* represent a difference between an impedance collected in the j^{th} round and an impedance collected in the (j-1)^{th} round; and *Z*_{*i*(*j*-1)} and *Z*_{*ii*(*j*-1)} represent impedance values collected in the (j-1)^{th} round.

Each round of collected data is calculated by using the foregoing formulas (1) to (3), to respectively obtain six pieces of power difference data, six pieces of temperature difference data, and 21 pieces of impedance difference data.

S42: Determine, after each round of collected data, whether the power data, the temperature data, and the impedance data all reach set target variation ranges ([P_{L}, P_{H}], [T_{L}, T_{H}], [Z_{L}, Z_{H}]) for the first time. Increase rates of the power data, the temperature data, and the impedance data are calculated if the power data, the temperature data, and the impedance data all reach the set target variation ranges for the first time. A next step is skipped if the power data, the temperature data, and the impedance data do not simultaneously reach the set target variation ranges or do not reach the set target variation ranges for the first time.

It is assumed that, in first collection, an initial power value is Pᵢₛ, an initial temperature value is Tᵢₛ, initial impedance values are Zᵢₛ and Zᵢᵢₛ, and an ablation duration is zero. In the j^{th} round of collected data, the power data Pij, the temperature data Tij, and the impedance data Zij and Ziij all reach the set target variation ranges, and the ablation duration is tⱼ. The increase rates of the parameter data are calculated as follows: ${Pin}_{ij} = \frac{\left({P}_{ij}-{P}_{is}\right)}{{t}_{j}}$ (4)
where *Pinᵢⱼ* is an increase rate of the power collected in the j^{th} round. ${Tin}_{ij} = \frac{\left({T}_{ij}-{T}_{is}\right)}{{t}_{j}}$ (5)
where *Tinᵢⱼ* is an increase rate of the temperature collected in the j^{th} round. ${Zin}_{ij} = \frac{\left({Z}_{ij}-{Z}_{is}\right)}{{t}_{j}} , and {Zin}_{iij} = \frac{\left({Z}_{iij}-{Z}_{iis}\right)}{{t}_{j}}$ (6)
where *Zinᵢⱼ* and *Zinᵢᵢⱼ* are increase rates of impedances collected in the j^{th} round.

The j^{th} round of collected data is calculated by using the foregoing formulas (4) to (6), to respectively obtain six pieces of power increase rate data, six pieces of temperature increase rate data, and 21 pieces of impedance increase rate data. It is to be noted that the increase rate data of the power, the temperature, and the impedance is calculated (only once) only when the power, the temperature, and the impedance all reach the set target variation ranges for the first time, and the data values are cited in later endpoint determination.

S43: Calculate, after each round of collected data, maximum values, minimum values, and maximum fluctuation ranges of the power data, the temperature data, and the impedance data in a current ablation process.

Firstly, a maximum value, a minimum value, and a maximum fluctuation range of the power data are calculated as follows: ${Pmax}_{ij} = {max}_{n = 1 , 2 … j} {P}_{in}$ (7) ${Pmin}_{ij} = {min}_{n = 1 , 2 … j} {P}_{in}$ (8) ${PD}_{ij} = {Pmax}_{ij} - {P}_{{min}_{ij}}$ (9) where *Pmaxᵢⱼ, Pminᵢⱼ,* and *PDᵢⱼ* are respectively the maximum value, the minimum value, and the maximum fluctuation range of the power after the j^{th} round of collected data in the current ablation process; and n represents a sequence number of each round of collected data, and *Pᵢₙ* represents a power value collected each time.

Each round of collected data is calculated by using the foregoing formulas (7) to (9), to respectively obtain six maximum power values, six minimum power values, and six maximum power fluctuation ranges.

Secondly, the maximum value, the minimum value, and the maximum fluctuation range of the temperature data are calculated as follows: ${Tmax}_{ij} = {max}_{n = 1 , 2 … j} {T}_{in}$ (10) ${Tmin}_{ij} = {min}_{n = 1 , 2 … j} {T}_{in}$ (11) ${TD}_{ij} = {Tmax}_{ij} - {Tmin}_{ij}$ (12) where *Tmaxᵢⱼ, Tminᵢⱼ,* and *TDᵢⱼ* are respectively the maximum value, the minimum value, and the maximum fluctuation range of the temperature after the j^{th} round of collected data in the current ablation process; and n represents a sequence number of each round of collected data, and *Tᵢₙ* represents a temperature value collected each time.

Each round of collected data is calculated by using the foregoing formulas (10) to (12), to respectively obtain six maximum temperature values, six minimum temperature values, and six maximum temperature fluctuation ranges.

Secondly, a maximum value, a minimum value, and a maximum fluctuation range of the impedance data are calculated as follows: ${Zmax}_{ij} = {max}_{n = 1 , 2 … j} {Z}_{in} , and {Zmax}_{iij} = {max}_{n = 1 , 2 … j} {Z}_{iin}$ (13) ${Zmin}_{ij} = {min}_{n = 1 , 2 … j} {Z}_{in} , and {Zmin}_{iij} = {min}_{n = 1 , 2 … j} {Z}_{iin}$ (14) ${ZD}_{ij} = {Zmax}_{ij} - {Zmin}_{ij} , and {ZD}_{iij} = {Zmax}_{iij} - {Zmin}_{iij}$ (15) where *Zmaxᵢⱼ* and *Zmaxᵢᵢⱼ, Zminᵢⱼ* and *Zminᵢᵢⱼ* , and *ZDᵢⱼ* and *ZDᵢᵢⱼ* are respectively maximum values, minimum values, and maximum fluctuation ranges of the impedances after a j^{th} round of collected data in the current ablation process; and n represents a sequence number of each round of collected data, and *Zᵢₙ* and *Zᵢᵢₙ* represent impedance values collected each time.

Each round of collected data is calculated by using the foregoing formulas (13) to (15), to respectively obtain 21 maximum impedance values, 21 minimum impedance values, and 21 maximum impedance fluctuation ranges.

S44: Calculate, after each round of collected data, standard deviations of the impedance data, the temperature data, and the power data in the current ablation process. ${Pstd}_{ij} = {std}_{n = 1 , 2 … j} {P}_{in}$ (16) ${Tstd}_{ij} = {std}_{n = 1 , 2 … j} {T}_{in}$ (17) ${Zstd}_{ij} = {std}_{n = 1 , 2 … j} {Z}_{in} , and {Zstd}_{iij} = {std}_{n = 1 , 2 … j} {Z}_{iin}$ (18) where *Pstdᵢⱼ, Tstdᵢⱼ, Zstdᵢⱼ,* and *Zstdᵢᵢⱼ* are respectively a standard deviation of a power, a standard deviation of a temperature, and a standard deviation of an impedance after a j^{th} round of collected data in the current ablation process; and n represents a sequence number of each round of collected data, and *Pᵢₙ*, *Tᵢₙ*, *Zᵢₙ*, and *Zᵢᵢₙ* represent a power value, a temperature value, and an impedance value collected each time.

Each round of collected data is calculated by using the foregoing formulas (16) to formula (18), to respectively obtain six pieces of power standard deviation data, six pieces of temperature standard deviation data, and 21 pieces of impedance standard deviation data.

In an embodiment of the present disclosure, 198 pieces of parameter data, referred to as processed data, in total are obtained by performing data processing of the foregoing step S41 to step S44 on each round of collected data, and 232 pieces of parameter data in total are obtained by adding 34 pieces of collected data.

S5: Determine an ablation endpoint according to the collected data of the power, the temperature, the impedance, and the ablation duration, and the processed data of the power, the temperature, and the impedance.

Each round of collected data and the processed data are respectively compared with respective set target values, to determine whether each piece of data reaches the set target value, and then determine whether an ablation endpoint is reached by using the following method.

The collected data and processed data obtained in each round of data collection are denoted as *Xₖ₋ⱼ,* a set target value of each parameter is denoted as *Yₖ,* and a set weight value of each parameter is denoted as *ωₖ,* where j represents a number of a count of one round of data collection, that is, a j^{th} data collection; and k represents numbers of collected data and processed data obtained by one round of data collection, where k=1, 2, 3, ..., or 232.

Determination and related calculation of the ablation endpoint are as follows:
S51: Calculate a valid value *rₖ₋ⱼ* of each piece of parameter data: ${r}_{k - j} = \left\{\begin{matrix}0 , & {X}_{k - j} < {Y}_{k} \\ 1 , & {X}_{k - j} \geq {Y}_{k}\end{matrix}\right.$ (19)

By means of calculation of the formula (19), when a collected data value of a particular parameter reaches (is greater than or equal to) a set target value, a valid value of the parameter is 1; otherwise, the valid value is 0.

S52: Calculate a total valid value *Nⱼ*: ${N}_{j} = {\sum }_{k = 1}^{232} {ω}_{k} \times {r}_{k - j}$ (20)

By means of calculation of the formula (20), set weights of the parameter data reaching the set target values are accumulated to obtain an overall valid value after each round of data collection.

S53: Accumulate a total qualified count Q and a maximum value Q1 of continuous qualified counts:
comparing the total valid value *Nⱼ* after each round of data collection with a set total valid value threshold M, denoting a current data collection as a qualified count when the total valid value *Nⱼ* after the data collection is greater than or equal to the total valid value threshold M, and accumulating the qualified count in the total qualified count Q. The maximum value Q1 of the continuous qualified counts is further counted and recorded.

Determination of an ablation endpoint: when the total qualified count Q obtained after one round of data collection reaches a set qualified count threshold, or the maximum value Q1 of the continuous qualified counts reaches a set continuous qualified count threshold, it is determined that a current ablation reaches an endpoint.

In an embodiment of the present disclosure, for different ablation areas, a set qualified count threshold range is 60 to 200, and a set continuous qualified count threshold is 40 to 120.

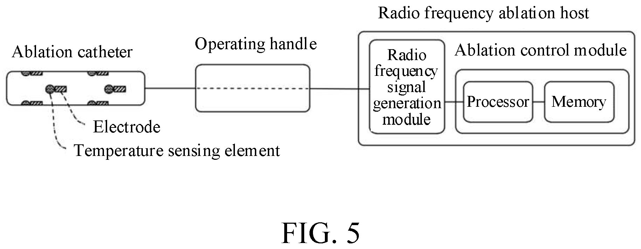

The above describes a method for determining an ablation endpoint of an intravascular nerve ablation according to the present disclosure in detail. Based on the method for determining an ablation endpoint, an embodiment of the present disclosure further provides a radio frequency ablation device. As shown in FIG. 5, the radio frequency ablation device includes an ablation catheter, an operating handle, and a radio frequency ablation host. The ablation catheter, the operating handle, and the radio frequency ablation host are sequentially connected. A plurality of electrodes and a plurality of temperature sensing elements are disposed at a front end of the ablation catheter. The radio frequency ablation host at least includes a radio frequency signal generation module and an ablation control module. The ablation control module is configured to control an entire ablation process and an ablation endpoint, and includes one or more processors and memories. The memories are coupled to the processors, and are configured to store one or more computer programs. When the one or more computer programs are executed by the one or more processors, the one or more processors implement the method for determining an ablation endpoint of an intravascular nerve ablation as described in the foregoing embodiment. The processor is configured to control an overall operation of the radio frequency ablation device, to complete all or some steps of the foregoing method for determining an ablation endpoint of an intravascular nerve ablation. A processor module may be a central processing unit (CPU), a graphics processing unit (GPU), a field programmable gate array (FPGA), an application-specific integrated circuit (ASIC), a digital signal processing (DSP) chip, or the like. The memory is configured to store various types of data to support operations of the radio frequency ablation device. The data may include, for example, instructions for any application or method for operating the ablation device, and data related to the application. A memory module may be implemented by any type of volatile or non-volatile storage devices or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, and a flash memory.

In conclusion, compared with the conventional art, according to the method for determining an ablation endpoint of an intravascular nerve ablation according to the embodiments of the present disclosure, operating parameters of a power, a temperature, an impedance, and an ablation duration at each electrode are cyclically collected, and a plurality of related operating parameters of the power, the temperature, and the impedance are obtained through data processing, to accurately and comprehensively determine an ablation progress and an ablation endpoint, thereby ensuring an ablation effect and ablation safety. Therefore, the method for determining an ablation endpoint of an intravascular nerve ablation according to this embodiment of the present disclosure has beneficial effects such as safety, accuracy, and optimization of an ablation effect.

It is to be noted that terms "first" and "second" are used merely for the purpose of description, and cannot be construed as indicating or implying relative importance or implying a quantity of indicated technical features. Therefore, features defined by "first" and "second" may explicitly or implicitly include one or more of the features. In the description of the present disclosure, "a plurality of" means two or more, unless otherwise limited explicitly and specifically.

The foregoing describes a method for determining an ablation endpoint of an intravascular nerve ablation and an ablation device according to the present disclosure in detail. For a person of ordinary skill in the art, any apparent change made to the present disclosure without departing from the essence of the present disclosure will constitute a violation of the patent right of the present disclosure and take corresponding legal responsibility.

## Claims

1. A method for determining an ablation endpoint of an intravascular nerve ablation, comprising the following steps:
(1) setting target values and target variation ranges of operating parameters, wherein the operating parameters at least comprise a power, a temperature, an impedance, and an ablation duration, related operating parameters of the power, the temperature, and the impedance, weights of the operating parameters, a total valid value threshold, a qualified count threshold, and a continuous qualified count threshold;
(2) sending an ablation catheter to a target ablation position and confirming good electrode contact;
(3) cyclically collecting operating parameter data at each electrode at set time intervals, comprising data of the power, the temperature, the impedance, and the ablation duration;
(4) performing corresponding calculation processing on each round of collected data from a second round of collected data, to obtain related processed data of the power, the temperature, and the impedance at each electrode, wherein the related processed data comprises differences, maximum values, minimum values, maximum fluctuation ranges, and standard deviations of the power, the temperature, and the impedance, and increase rates of the power, the temperature, and the impedance when reaching the set target variation ranges for the first time; and
(5) determining an ablation endpoint according to the collected data of the power, the temperature, the impedance, and the ablation duration, and the processed data of the power, the temperature, and the impedance.

2. The method for determining an ablation endpoint of an intravascular nerve ablation according to claim 1, wherein in step (1), the related operating parameters comprise differences and increase rates between radio frequency powers, ablation temperatures, and tissue impedances in data continuously collected in two rounds, and a maximum value, a minimum value, and a standard deviation value of the radio frequency power, the ablation temperature, and the tissue impedance in currently collected data.

3. The method for determining an ablation endpoint of an intravascular nerve ablation according to claim 1, wherein step (3) comprises the following substeps:
(31) in a monitoring mode, collecting impedance data between each electrode and a body surface neutral electrode, collecting impedance data between every two electrodes, and collecting temperature data at each electrode; and
(32) in a radio frequency mode, collecting the power data and the ablation duration data output to each electrode.

4. The method for determining an ablation endpoint of an intravascular nerve ablation according to claim 1, wherein in step (4), a power difference *Pdᵢⱼ,* a temperature difference *Tdᵢⱼ,* and impedance differences *Zdᵢⱼ* and *Zdᵢᵢⱼ* between each round of collected data and a previous round of collected data are calculated as follows: ${Pd}_{ij} = {P}_{ij} - {P}_{i \left(j-1\right)}$ ${Td}_{ij} = {T}_{ij} - {T}_{i \left(j-1\right)}$ ${Zd}_{ij} = {Z}_{ij} - {Z}_{i \left(j-1\right)}$ ${Zd}_{iij} = {Z}_{iij} - {Z}_{ii \left(j-1\right)}$ wherein j represents a j^{th} round of data collection, and j-1 represents a previous round of data collection; *Pij* and *Tij* represent power data and temperature data in the j^{th} round of data collection, and *Zij* and *Ziij* represent impedance data in the j^{th} round of data collection;*P*_{*i*(*j*-1)} and *T*_{*i*(*j*-1)} represent power data and temperature data in a (j-1)^{th} round of data collection, and *Z*_{*i*(*j*-1)} and *Z*_{*ii*(*j*-1)} represent impedance data in the (j-1)^{th} round of data collection; and *i* represents an electrode number, and *ii* represents a number between two electrodes.

5. The method for determining an ablation endpoint of an intravascular nerve ablation according to claim 1, wherein in step (4), after each round of collected data, whether power data, temperature data, and impedance data all reach the set target variation ranges for the first time is determined; increase rates of the power data, the temperature data, and the impedance data are calculated if the power data, the temperature data, and the impedance data all reach the set target variation ranges for the first time; a next step is skipped if the power data, the temperature data, and the impedance data do not simultaneously reach the set target variation ranges or do not reach the set target variation ranges for the first time;
after a j^{th} round of collected data, and when the power, the temperature, and the impedance all reach the set target variation ranges, a power increase rate *Pinᵢⱼ,* a temperature increase rate *Tinᵢⱼ,* and impedance increase rates *Zinᵢⱼ* and *Zinᵢᵢⱼ* are calculated as follows: ${Pin}_{ij} = \frac{\left({P}_{ij}-{P}_{is}\right)}{{t}_{j}}$ ${Tin}_{ij} = \frac{\left({T}_{ij}-{T}_{is}\right)}{{t}_{j}}$ ${Zin}_{ij} = \frac{\left({Z}_{ij}-{Z}_{is}\right)}{{t}_{j}}$ ${Zin}_{iij} = \frac{\left({Z}_{iij}-{Z}_{iis}\right)}{{t}_{j}}$
wherein j represents a j^{th} round of data collection, *Pij* and *Tij* represent the power data and the temperature data in the j^{th} round of data collection, *Zij* and *Ziij* represent the impedance data in the j^{th} round of data collection, and *tⱼ* is an ablation duration in the j^{th} round of data collection; *i* represents an electrode number, and *ii* represents a number between two electrodes; and *Pᵢₛ* and *Tᵢₛ* represent an initial power value and an initial temperature value obtained in first data collection, and *Zᵢₛ* and *Zᵢᵢₛ* represent initial impedance values obtained in the first data collection.

6. The method for determining an ablation endpoint of an intravascular nerve ablation according to claim 1, wherein in step (4), after each round of collected data, a maximum value *Pmaxᵢⱼ,* a minimum value *Pminᵢⱼ,* and a maximum fluctuation range *PDᵢⱼ* of the power in a current ablation process are calculated as follows: ${Pmax}_{ij} = {max}_{n = 1 , 2 … j} {P}_{in}$ ${Pmin}_{ij} = {min}_{n = 1 , 2 … j} {P}_{in}$ ${PD}_{ij} = {Pmax}_{ij} - {Pmin}_{ij}$
after each round of collected data, a maximum value *Tmaxᵢⱼ,* a minimum value *Tminᵢⱼ,* and a maximum fluctuation range *TDᵢⱼ* of the temperature in the current ablation process are calculated as follows: ${Tmax}_{ij} = {max}_{n = 1 , 2 … j} {T}_{in}$ ${Tmin}_{ij} = {min}_{n = 1 , 2 … j} {T}_{in}$ ${TD}_{ij} = {Tmax}_{ij} - {Tmin}_{ij}$
after each round of collected data, maximum values *Zmaxᵢⱼ* and *Zmaxᵢᵢⱼ,* minimum values *Zminᵢⱼ* and *Zminᵢᵢⱼ,* and maximum fluctuation ranges *ZDᵢⱼ* and *ZDᵢᵢⱼ* of the impedance in the current ablation process are calculated as follows: ${Zmax}_{ij} = {max}_{n = 1 , 2 … j} {Z}_{in}$ ${Zmax}_{iij} = {max}_{n = 1 , 2 … j} {Z}_{iin}$ ${Zmin}_{ij} = {min}_{n = 1 , 2 … j} {Z}_{in}$ ${Zmin}_{iij} = {min}_{n = 1 , 2 … j} {Z}_{iin}$ ${ZD}_{ij} = {Zmax}_{ij} - {Zmin}_{ij}$ ${ZD}_{iij} = {Zmax}_{iij} - {Zmin}_{iij}$
wherein j represents a j^{th} round of data collection, n represents a sequence number of each round of collected data, *Pᵢₙ* represents a power value in data collected each time, *Tᵢₙ* represents a temperature value in the data collected each time, *Zᵢₙ* and *Zᵢᵢₙ* represent impedance values collected each time, *i* represents an electrode number, and *ii* represents a number between two electrodes.

7. The method for determining an ablation endpoint of an intravascular nerve ablation according to claim 1, wherein in step (4), after each round of collected data, a power standard deviation *Pstdᵢⱼ ,* a temperature standard deviation *Tstdᵢⱼ ,* and impedance standard deviations *Zstdᵢⱼ* and *Zstdᵢᵢⱼ* in a current ablation process are calculated as follows: ${Pstd}_{ij} = {std}_{n = 1 , 2 … j} {P}_{in}$ ${Tstd}_{ij} = {std}_{n = 1 , 2 … j} {T}_{in}$ ${Zstd}_{ij} = {std}_{n = 1 , 2 … j} {Z}_{in}$ ${Zstd}_{iij} = {std}_{n = 1 , 2 … j} {Z}_{iin}$ wherein j represents a *j^{th}* round of data collection, *n* represents a sequence number of each round of collected data, *Pᵢₙ* represents a power value in data collected each time, *Tᵢₙ* represents a temperature value in the data collected each time, *Zᵢₙ* and *Zᵢᵢₙ* represents impedance values collected each time, *i* represents an electrode number, and *ii* represents a number between two electrodes.

8. The method for determining an ablation endpoint of an intravascular nerve ablation according to claim 1, wherein step (5) comprises the following substeps:
(51) calculating a valid value *rₖ₋ⱼ* of each piece of parameter data after each round of collected data: ${r}_{k - j} = \left\{\begin{matrix}0 , & {X}_{k - j} < {Y}_{k} \\ 1 , & {X}_{k - j} \geq {Y}_{k}\end{matrix}\right.$
wherein j represents a *j^{th}* round of data collection, *k* represents numbers of collected data and processed data obtained by one round of data collection, k=1, 2, 3, ..., *Xₖ₋ⱼ* represents specific collected data or processed data, *ωₖ* represents a set weight corresponding to the specific collected data or processed data, and *Yₖ* represents a set target value corresponding to the specific collected data or processed data;
(52) calculating a total valid value *Nⱼ*: ${N}_{j} = {\sum }_{k = 1}^{232} {ω}_{k} \times {r}_{k - j}$
wherein 232 is a total quantity of the collected data and processed data obtained in one round of data collection; and
(53) accumulating a total qualified count Q and a maximum value Q1 of continuous qualified counts:
comparing the total valid value after each round of data collection with a set total valid value threshold, denoting a current data collection as a qualified count when the total valid value after the data collection is greater than or equal to the total valid value threshold, and accumulating the qualified count in the total qualified count (Q); and simultaneously, recording the maximum value (Q1) of the continuous qualified counts.

9. The method for determining an ablation endpoint of an intravascular nerve ablation according to claim 8, wherein
when the total qualified count obtained after one round of data collection reaches a set qualified count threshold, or the maximum value of the continuous qualified counts reaches a set continuous qualified count threshold, it is determined that a current ablation reaches an endpoint.

10. A radio frequency ablation device, comprising an ablation catheter, an operating handle, and a radio frequency ablation host, wherein the ablation catheter, the operating handle, and the radio frequency ablation host are sequentially connected, wherein
a plurality of electrodes and a plurality of temperature sensing elements are disposed at a front end of the ablation catheter;
the radio frequency ablation host at least comprises a radio frequency signal generation module and an ablation control module; and
the ablation control module is configured to control an entire ablation process and an ablation endpoint, the ablation control module comprises a processor and a memory, and the processor is coupled to the memory, wherein the memory is configured to store a computer program, and the processor is configured to execute the computer program stored in the memory, to perform the method for determining an ablation endpoint of an intravascular nerve ablation according to any one of claims 1 to 9.
